# EUROPEAN PATENT APPLICATION

(11) **EP 4 616 884 A1**
(43) Date of publication of application: **17.09.2025**
(21) Application number: 24205579.6
(22) Date of filing: 09.10.2024
(51) Int. Cl.: A61M 5/24

(54) **LOADING MECHANISM FOR A DRUG DELIVERY DEVICE**

(71) Applicant: Ypsomed AG, 3401 Burgdorf (CH)
(72) Inventor: Loser, Peter, 3037 Herrenschwanden (CH); Bernhard, Mario, 3400 Burgdorf (CH); Wegner, Stephan, 4051 Basel (CH); Shepherd, David, Edinburgh, EH12 7HW (GB); Fischbacher, Peter Alastair, Edinburgh, EH11 2LX (GB); McLusky, James Donald, Edinburgh, EH6 4QN (GB)

(57) **Abstract**

The present invention relates to a loading mechanism for a drug delivery device for dispensing a liquid drug from a reservoir through a needle, the loading mechanism comprising a drive unit and a reservoir unit adapted to hold the reservoir and releasably attachable to the drive unit. The drive unit comprises a drive unit housing defining a longitudinal axis, a plunger movable inside the drive unit housing in a dispensing direction to dispense the liquid drug and a mechanical drive, preferably a compression spring, arranged inside the drive unit housing for moving, when loaded, the plunger in the dispensing direction. The reservoir unit comprises a reservoir unit housing. For loading the drug delivery device, the reservoir unit is insertable into the drive unit housing from a distal end of the drive unit. According to the invention the reservoir unit housing comprises an abutment surface that is adapted to contact the plunger and to push the plunger in a proximal direction to load the mechanical drive, preferably to compress or re-set the compression spring, while the reservoir unit is inserted into the drive unit housing.

## Description

### FIELD OF THE INVENTION

The present invention relates to drug delivery devices or medicament delivery devices for injecting, delivering, administering, infusing or dispensing substances and/or liquids such as insulin, hormone preparations or vaccines. It departs from a drug delivery device comprising a preferably reusable drive unit and a preferably disposable reservoir unit configured for releasable attachment to the drive unit.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a stopper in a container barrel, wherein the drive spring may have to be charged or strained manually prior to injection of a dose. A manually powered delivery drive requires a user to manually provide the energy to move the stopper, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the stopper manually or by pressing a release button of an automatic injection device. Automatic injection devices may comprise an electronic dose dial mechanism to automatically set a dose.

Also known is the use of autoinjectors with prefilled syringes. Autoinjectors usually comprise a body for housing a syringe as well as a drive mechanism in order to move the stopper of the syringe upon actuation of the autoinjector. The drive mechanism typically comprises a source of drive, such as an electric motor or a mechanical spring for moving a transfer element, for example a plunger rod, which acts on the stopper of the syringe. The prefilled syringe has a needle or cannula that is permanently attached to a first end of a syringe barrel or reservoir that is sealed at the opposing end by the stopper.

For safety and hygiene reasons it is desirable that the needle does not protrude from a housing of the autoinjector except for the time when the needle is used for injection of a medicament. Thus, either the autoinjector moves the needle out of the housing for the injection and back into the housing after injection or the autoinjector includes a needle cover which may be moved to unsheathe the needle for injection, and which may be moved back to a needle covering position after the injection.

The majority of autoinjectors are configured as single use devices which incorporate both the syringe and the drive mechanism in the same housing. Such devices are usually disposable for hygiene reasons. Disposable autoinjectors comprising an electric actuator or an electronic control require a source of energy which is usually in the form of a battery. However, in this case, the autoinjectors should not be disposed in the regular waste but have to be subjected to special disposal or to recycling, which is an additional burden to the patient. Further, disposing a battery, motor and/or electronics after a single use is a waste of resources and increases the costs of the therapy. Disposable autoinjectors comprising a mechanical drive, like a compression spring, are easier to dispose than autoinjectors with an electric drive, however it is also a waste of resources to dispose the compression spring after a single use, which also increases the costs of the therapy.

In order to account for a need to handle and dispose the autoinjector parts differently semi-reusable autoinjectors have been developed. Such autoinjectors typically comprise a reusable drive unit and a disposable syringe unit which may be releasably coupled or attached to the drive unit. The drive unit usually includes the drive mechanism and electronics whereas the syringe unit includes the syringe with the needle and a needle cover sleeve. The user can thus discard the syringe unit when it is empty or after use and can load the drive unit with a new syringe unit for a new upcoming injection. A big problem for semi-reusable mechanical autoinjectors is that the compression spring has to be recompressed or re-loaded after each injection, which can require a high force and accuracy, and can therefore be challenging for the user, especially for older patients.

EP 2618870 A1 discloses a semi-reusable autoinjector comprising a reusable drive unit with a mechanical drive mechanism and a disposable syringe unit. When the syringe unit is inserted in the drive unit, the syringe unit rotates a winder element that is operationally connected to an input transmission train for charging the mechanical drive mechanism.

WO 18091916 A1 discloses a semi-reusable autoinjector with a reusable drive unit and a disposable reservoir unit that can be releasably connected to the drive unit. Before the reservoir unit is connected to the drive unit, a compression spring for driving a plunger to expel the medicament has to be loaded by inserting the drive unit in a special reloading station that compresses the spring.

US 2016/354556 A1 discloses a semi-reusable autoinjector comprising a reusable driving assembly with a compression spring to move a plunger and a disposable cassette. The cassette is removably insertable into the driving assembly at the forward injection end and is removably retained within the driving assembly by snap fit engagement. Before the cassette is inserted into the driving assembly, the compression spring has to be charged by pressing the driving assembly against a flat surface, like a desk or a wall.

WO 14029621 A1 discloses a semi-reusable autoinjector comprising a front housing and a rear housing, releasably connectable to each other, and a replaceable container assembly. The rear housing comprises a reloadable drive mechanism for acting on a stopper of the container assembly for expelling a medicament. A needle shield remover that is used to remove a needle shield of the container assembly can also be used to reload the drive mechanism. For reloading the drive mechanism, a user can insert the needle shield remover into the rear housing to load the mechanical drive before the container assembly is inserted into the rear housing and before the front housing and the rear housing are connected.

These known semi-reusable autoinjectors each need a plurality of different components to be able to reload the mechanical drive before an injection, which can increase the costs of these autoinjectors. Often the loading process also requires a plurality of work steps that have to be carried out in a specific order, which can take a lot of time and can be hard to remember for some users.

CN 217489449 U discloses a semi-reusable autoinjector comprising a drive unit and a detachable syringe unit. During the insertion, the stopper of the syringe contacts the plunger and moves the plunger to a retracted position to load the mechanical drive. A drawback of this injection device is that the stopper can move with respect to the syringe body to be closer to the injection needle, when the force of the mechanical drive is higher than the friction between the stopper and the syringe body. This movement of the stopper during the insertion can already dispense a portion of the medicament while the syringe unit is inserted into the drive unit, hence before the actual injection is started.

### DESCRIPTION OF THE INVENTION

It is an objective of the present invention to provide a drug delivery device with a mechanical drive that allows a user to load the drug delivery device by loading or compressing the mechanical drive in an easy and reliable way before each injection.

This objective is achieved by the features of the independent claims. Preferred embodiments are evident from the dependent claims.

The invention relates to a loading mechanism for a drug delivery device for dispensing a liquid drug from a reservoir through a needle.

The loading mechanism comprises a drive unit and a reservoir unit adapted to hold the reservoir and releasably attachable or connectable to the drive unit. With the loading mechanism, the reservoir unit can be inserted into the drive unit before each injection. The drug delivery device is preferably an injection device and more preferably an autoinjector, that is adapted to dispense the whole content of the reservoir in a single injection stroke. Most preferably, the drug delivery device is a semi-disposable autoinjector with a reusable drive unit and a disposable reservoir unit. After each injection the empty reservoir unit is disposed and replaced by a new disposable reservoir unit. The reservoir may be a prefilled syringe having a needle or cannula that is permanently attached to a first end of a syringe barrel that is sealed at the opposing end by a stopper. Alternatively, the reservoir may be a cartridge with a connecting portion at the distal end adapted to be connected to an injection needle before the injection.

The drive unit of the loading mechanism comprises a drive unit housing defining a longitudinal axis and a plunger movable inside the drive unit housing in a dispensing direction to dispense the liquid drug. The plunger can also be referred to as piston rod or drive element. The longitudinal axis defines a longitudinal direction of the drive unit. The longitudinal axis of the drive unit is also considered as longitudinal axis of the drug delivery device. The dispensing direction can preferably be the longitudinal direction and more preferably a distal direction. The drive unit housing can comprise only one part with a sleeve structure. The drive unit housing can also comprise two or more parts that together form a sleeve structure. In this alternative, the two or more drive unit housing parts can be connected mechanically, for example with screws or latches, and/or they can be bonded to each other, for example by gluing or welding. The drive unit further comprises a mechanical drive arranged inside the drive unit housing for moving, when loaded with the loading mechanism, the plunger in the dispensing direction. Preferably, the mechanical drive is a compression spring. For loading, the mechanical drive is compressed in a proximal direction, hence a distal end of the mechanical drive is moved in the proximal direction while the proximal end of the mechanical drive is fixed.

For loading the drug delivery device, the reservoir unit is insertable into the drive unit housing from a distal end of the drive unit. Preferably the drive unit is inserted by a user prior to an injection to assemble the drug delivery device and to prepare the drug delivery device for the injection. This means the drug delivery device is not fully assembled by the manufacturer before the reservoir unit and the drive unit are delivered to the user as individual components. The drive unit housing can comprise a first connection element and the reservoir unit housing can comprise a second connection element, that are adapted to attach or connect the reservoir unit to the drive unit in a predefined end position within the drive unit housing. The first connection element and the second connection element have to be adapted to be able to provide the connection between the reservoir unit and the drive unit against the force of the loaded mechanical drive and eventually against the force of removing a protective cap from the reservoir unit after attaching the reservoir unit to the drive unit and before the injection is started.

The reservoir unit of the loading mechanism comprises a reservoir unit housing. In the present invention, the term reservoir unit housing refers to the outermost component of the reservoir unit seizable or grippable by a user, with other words the outermost component of the reservoir unit that the user grips while inserting the reservoir unit into the drive unit. The reservoir unit housing also defines a longitudinal axis and a longitudinal direction of the reservoir unit, that coincides with the longitudinal axis and the longitudinal direction of the drive unit, when the reservoir unit is inserted into the drive unit housing. Preferably, the reservoir unit housing is adapted to hold the reservoir. However, in other alternatives the reservoir unit can further comprise a reservoir holder arranged inside the reservoir unit housing that holds the reservoir. Preferably, after assembly of the reservoir unit, the reservoir is fixed or non-movably arranged inside the reservoir unit housing or the reservoir holder. That means during an injection the reservoir does not move relative to the reservoir unit housing or the reservoir holder and hence also does not move relative to the drive unit housing. This means that the user has to insert or penetrate the injection needle into the injection site by a manual insertion force.

According to the invention, the reservoir unit housing comprises an abutment surface that is adapted to contact the plunger and to push the plunger in a proximal direction to load the mechanical drive, while the reservoir unit is inserted into the drive unit housing. Loading the mechanical drive preferably means to compress or re-set the compression spring. Hence, the plunger is moved, preferably in a linear movement, in the proximal direction by the abutment surface of the reservoir unit housing and thereby compresses the mechanical drive by pushing the distal end of the mechanical drive in the proximal direction. With the inventive loading mechanism, the reservoir unit can be inserted into the drive unit housing and at the same time respectively in the same step the mechanical drive can be loaded. Preferably, the mechanical drive is exclusively loaded while the reservoir unit is inserted into the drive unit housing. This means that before the reservoir unit is inserted, the mechanical drive is unloaded or unstressed and at the time the reservoir unit reaches the predefined end position within the drive unit, the mechanical drive is fully loaded or compressed, so that no further loading is necessary before the injection. Preferably, the abutment surface is located on a proximal end surface of the reservoir unit housing that faces the plunger, when the reservoir unit is inserted into the drive unit.

An advantage of the loading mechanism according to the invention is that the user has to execute only one step, namely inserting the reservoir unit into the drive unit housing, to load the mechanical drive and to attach the reservoir unit to the drive unit at the same time. Hence, after one single assembly step the drug delivery device is fully assembled and ready to use. Another advantage of the inventive loading mechanism is that it is easier for the user to load the mechanical drive as the user directly grips the reservoir unit housing that is used to push the plunger and is not deformable, hence the user can directly control the force applied on the plunger. In addition, the stopper of the reservoir does not come in contact with the drive unit, in particular the plunger, while the reservoir unit is inserted into the drive unit, so that there is no risk that a portion of the medicament is dispensed before the actual injection, as no force is applied on the stopper.

In alternative embodiments, the abutment surface may also be part of a needle cover of the reservoir unit or any other component of the reservoir unit different than the reservoir itself., such as the reservoir holder.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The term "distal" is meant to refer to the direction or the end of the drug delivery device carrying an injection needle or an injection cannula, whereas the term "proximal" is meant to refer to the opposite direction or end pointing away from the needle or cannula.

The term "injection device" or "injector" refers to a device that is removed from the injection site after each medication event or drug delivery process, whereas the term "infusion device" refers to a device with a cannula or needle that remains in the skin of the patient for a prolonged period of time, for example, several hours.

In the claims, the word "comprising" or "comprises" does not exclude other elements or steps, and the indefinite article "a" or "an" does not exclude a plurality. For example, "an arm" does not exclude the fact that there may be two or more arms that functionally or structurally fulfill the purpose of "an arm". The mere fact that certain elements or steps are recited in distinct claims shall not preclude the existence of further meaningful combinations of these elements or steps.

In a preferred embodiment, the plunger comprises an inner rod section adapted to contact and move a stopper of the reservoir for dispensing the liquid drug and a plunger arm, arranged radially offset from the inner rod section adapted to contact the abutment surface. Preferably, the plunger comprises two plunger arms arranged opposite to each other. This design of the plunger makes it possible to use the abutment surface of the reservoir unit housing to move the plunger and to load the mechanical drive in an easy and reliable way. Due to the additional plunger arm, the inner rod section can only be used to move the stopper during the injection and is not used to move the plunger in the proximal direction to load the mechanical drive while the reservoir unit is inserted into the drive unit. Therefore, there is no risk that the stopper is pushed in the distal direction with respect to the reservoir, so that the medicament is not dispensed before the injection starts. Preferably, the inner rod section and the plunger arm are connected at a proximal end of the plunger arm. Preferably, the inner rod section and the plunger arm are integrally formed, which allows to manufacture the plunger according to the preferred embodiment in an easy and cheap way for example by injection molding.

Preferably, the plunger arm comprises a plunger clip that is elastically deformable in a radial direction and adapted to hold the plunger in an initial position against a bias of the loaded mechanical drive and to release the plunger in a deformed position. This allows to fix the plunger relative to the drive unit housing and to release the plunger to start an injection by means of the same plunger arm that contacts the plunger during the insertion of the reservoir unit into the drive unit, so that no further arms or projections or other features are needed to fix and release the plunger. This allows a simple design of the plunger. In the initial position the plunger clip can be closer to the longitudinal axis than in the deformed position. With other words, the plunger clip can be deformed outwards in the radial direction to reach the deformed position.

Preferably, the plunger arm comprises a projection on an inner surface of the plunger arm that is adapted to be contacted by the abutment surface of the reservoir unit housing. Due to the projection on the inner surface of the plunger arm, the reservoir unit housing with the abutment surface to move the plunger overlap with the plunger arm in the longitudinal direction, so that the dimensions of the drug delivery device in the longitudinal direction can be reduced. In alternatives with two or more plunger arms, the reservoir unit can be inserted between or inside the plunger arms. Most preferably, the projection is located on the plunger clip that is also used to fix and release the plunger, so that the plunger clip with the projection can realize two different functions, namely releasing the plunger to start the injection and loading the mechanical drive during insertion of the reservoir unit. Hence, no further features of the plunger are necessary to realize those two functions.

Preferably, the reservoir unit housing is engaged with the drive unit housing when inserted until a predefined end position within the drive unit housing, and the plunger is held by means of the projection abutting the abutment surface. An engagement between the reservoir unit housing and the drive unit housing can preferably be provided by latching the reservoir unit housing to the drive unit housing or by screwing the reservoir unit into the drive unit housing. The engagement can for example be realized by the first connection element of the drive unit housing and the second connection element of the reservoir unit housing. Due to the engagement of reservoir unit housing with the drive unit housing the reservoir unit cannot move in the distal direction relative to the drive unit, so that the plunger can also not move in the distal direction as long as the projection abuts the abutment surface of the reservoir unit housing. To release the plunger in order to start an injection, the projection can be separated from the abutment surface, for example by deforming the plunger arm or the plunger clip.

Preferably, for releasing the plunger, the plunger clip is deformable by a needle cover or cover member of the reservoir unit. The needle cover is movable along the longitudinal axis and guided by the reservoir unit housing. Preferably, the needle cover can be movable inside the reservoir unit housing, with other words be enclosed by the reservoir unit housing. The needle cover may be implemented as shield or sleeve or sleeve-shaped element adapted to cover or envelop the needle of the reservoir mounted inside the reservoir unit housing. The movement of the needle cover allows to switch between a safety state in which the needle is covered and does not protrude from the reservoir unit and a retracted injection state in which the needle is uncovered and protrudes from the reservoir unit. The needle cover is adapted to deform the plunger clip in the injection state, meaning that the injection can be started by pressing the needle cover against the skin at the injection site which pushes the needle cover in the proximal direction relative to the reservoir unit housing to the retracted position. To deform the plunger clip, the needle cover can comprise a protrusion, preferably on an outer surface of the needle cover, that is adapted to contact the plunger clip, preferably the protrusion on the plunger clip that is in contact with the abutment surface of the reservoir unit housing. In addition to the deformation of the plunger clip in the radial direction, the movement of the needle cover can also cause the plunger clip and hence the plunger to make a micro movement in the proximal direction. This micro movement also causes a further micro compression of the mechanical drive before the mechanical drive is released and moves in the distal direction to dispense the medicament.

In a preferred embodiment, the reservoir unit is insertable into the drive unit housing by a linear movement along the longitudinal axis, hence in a longitudinal direction. Preferably, the reservoir unit can also be separated from the drive unit housing by a linear movement along the longitudinal axis. This makes it particularly easy to insert the reservoir unit.

Preferably, the plunger clip is located on a distal end of the plunger arm and the projection is located on a free proximal end of the plunger clip. Hence, the distal end of the plunger clip connects the plunger clip to the plunger arm.

In a preferred embodiment, the reservoir unit is insertable into the drive unit housing by a turning movement around the longitudinal axis, preferably by screwing the reservoir unit into the drive unit housing. Preferably, the reservoir unit can also be separated from the drive unit housing by a turning movement around the longitudinal axis, preferably by screwing. The advantage of this preferred embodiment is that due to the force ratio resulting of the transmission of the rotational movement of the reservoir unit to a longitudinal movement of the plunger, a smaller force exerted by the user is sufficient to load the mechanical drive.

Preferably, the plunger clip is located on a distal end of the plunger arm and the projection is located on a free distal end of the plunger clip. Hence, the proximal end of the plunger clip connects the plunger clip to the plunger arm.

The invention also relates to a reservoir unit for an inventive loading mechanism as described above.

The reservoir unit comprises the reservoir unit housing, the reservoir and a needle cover. The needle cover or cover member is movable along the longitudinal axis and guided by the reservoir unit housing. Preferably, the needle cover is a needle cover sleeve. The movement of the needle cover allows to switch between a safety state in which the needle is covered and does not protrude from the reservoir unit and an injection state in which the needle is uncovered and protrudes from the reservoir unit. The reservoir unit housing of the inventive reservoir unit comprises an outer sleeve section and two inner reservoir arms arranged inside the outer sleeve section.

According to the invention, the reservoir unit is adapted to be assembled in the following way. In a first assembly step, the needle cover is insertable into the reservoir unit housing from a distal end of the reservoir unit housing, wherein the needle cover is insertable between the reservoir arms and the outer sleeve section. In a second assembly step, after the needle cover is inserted, the reservoir is insertable into the reservoir unit housing from a proximal end of the reservoir unit housing, Hence, from the other side of the reservoir unit housing than the needle cover. The reservoir is insertable inside the reservoir arms, hence the reservoir is enclosed by the reservoir arms and enclosed by the needle cover. This means the reservoir is the last component to be inserted into the reservoir unit housing. In a third assembly step, after the reservoir is inserted, the needle cover is movable in the distal direction from a first position in which the reservoir arms are free to move in a radial direction to a second position in which the needle cover prevents the reservoir arms from moving in the radial direction, so that the reservoir is fixed against distal movement inside the reservoir arms.

An advantage of the inventive reservoir unit is that the reservoir unit only comprises three components including the reservoir or two components excluding the reservoir, and eventually excluding a protective cap of the reservoir unit. To achieve this, the reservoir unit housing is adapted to hold the reservoir with the reservoir arms and to be gripped by a user at the outer sleeve section. This makes it possible to provide a reservoir unit without a separate syringe holder. Due to this, the manufacturing, the assembly, the handling and the storage of the reservoir unit is simplified compared to reservoir units with more components. A reduced number of components can thus help to reduce the overall production costs and to reduce the complexity of the reservoir unit. Besides that, the number of discarded components and thus the waste of the drug delivery device can be reduced which is advantageous from an environmental perspective. Another advantage of the inventive reservoir unit is that the reservoir can be fixed by simply moving the needle cover in the distal direction, so that there is no need for separate fixing means to fix the reservoir inside the reservoir unit housing. This also enables an easier assembly of the reservoir unit and a simpler design of the individual components.

Between the first assembly step and the second assembly step, a protective cap to cover the needle and if necessary to remove a needle shield of the reservoir can be connected to the reservoir unit housing from the distal end of the reservoir unit housing. If the cap is provided, the needle shield is pushed inside the protective cap and attached to the protective cap during the second assembly state, when the reservoir is inserted into the reservoir unit housing. In this alternative, the reservoir unit comprises four components including the reservoir or three components excluding the reservoir. Also in this alternative, the reservoir is the last component to be assembled.

In a preferred embodiment, the reservoir arms each comprise a protrusion facing the outer sleeve section, and the needle cover comprises two recesses adapted to receive the protrusions in the first position of the needle cover. In the second position of the needle cover, the recesses are spaced apart from the protrusions in the distal direction, so that the protrusions abut the inner surface of the needle cover and hence cannot move in the radial direction. This provides an easy and cheap possibility to fix the reservoir inside the reservoir unit housing.

Preferably, the protrusions are located near a distal end of the reservoir arms. By this the possible movement of the reservoir arms in the first position of the needle cover is as big as possible due to the big distance between the reservoir arms and the proximal end of the reservoir unit housing, so that the reservoir can easily be inserted in the reservoir arms.

The invention also relates to a drug delivery device comprising an inventive loading mechanism as described above. Preferably, the drive unit housing, the reservoir unit housing and/or the plunger are injection-molded thermoplastic plastic parts. In a preferred embodiment, the drug delivery device comprises an inventive reservoir unit as described above. Preferably, the needle cover is an injection-molded thermoplastic plastic part. The injection-molded parts allow for a cost-effective production of the reservoir unit parts, in particular in case of a high-volume production.

### BRIEF DESCRIPTION OF THE DRAWINGS

The subject matter of the invention will be explained in more detail in the following text with reference to preferred example embodiments which are illustrated in the attached drawings, in which depict:
- Fig. 1: a first example embodiment of an inventive drug delivery device before the reservoir unit is inserted into the drive unit;
- Fig. 2: the drug delivery device of fig. 1 after the reservoir unit is inserted into the drive unit;
- Fig. 3: an exploded view of the drug delivery device of fig. 1 and 2;
- Fig. 4: an exploded view of the reservoir unit of the drug delivery device of fig. 1 to 3;
- Fig. 5: a sectional view of the drug delivery device of fig. 1 to 3 while the reservoir unit is inserted into the drive unit;
- Fig. 6: the drug delivery device of fig. 1 to 5 after the reservoir unit is inserted into the drive unit;
- Fig. 7: a sectional view of the drug delivery device of fig. 6 along line VII - VII in fig. 6;
- Fig. 8: the drug delivery device of fig. 1 to 7 after the reservoir unit is inserted into the drive unit;
- Fig. 9: a sectional view of the drug delivery device of fig. 8 along line IX - IX in fig. 8;
- Fig. 10: a perspective view of the reservoir unit housing of the drug delivery device of fig. 1 to 9;
- Fig. 11: a first example embodiment of an inventive reservoir unit before assembly;
- Fig. 12: the reservoir unit of fig. 11 after the needle cover is inserted into the reservoir unit housing;
- Fig. 13: the reservoir unit of fig. 11 and 12 after the cap is attached to the reservoir unit housing;
- Fig. 14: the reservoir unit of fig. 11 to 13 after the reservoir is inserted into the reservoir unit housing;
- Fig. 15: the reservoir unit of fig. 11 to 14 after the needle cover is moved in the distal direction;
- Fig. 16: detail XVI of fig. 13 in an enlarged view;
- Fig. 17: detail XVII of fig. 15 in an enlarged view;
- Fig. 18: a second example embodiment of an inventive drug delivery device before the reservoir unit is inserted into the drive unit;
- Fig. 19: the drug delivery device of fig. 18 after the reservoir unit is inserted into the drive unit;
- Fig. 20: an exploded view of the drug delivery device of fig. 18 and 19;
- Fig. 21: an exploded view of the reservoir unit of the drug delivery device of fig. 18 to 20;
- Fig. 22: a sectional view of the drug delivery device of fig. 18 to 20 while the reservoir unit is inserted into the drive unit;
- Fig. 23: a sectional view of the drive unit of the drug delivery device of fig. 18 to 22 in an initial state;
- Fig. 24: a perspective view of the plunger of the drive unit of fig. 23;
- Fig. 25: a sectional view of the reservoir unit of the drug delivery device of fig. 18 to 22 in an initial state;
- Fig. 26: a perspective view of the reservoir unit housing of the reservoir unit of fig. 25;
- Fig. 27: a second example embodiment of an inventive reservoir unit before assembly;
- Fig. 28: the reservoir unit of fig. 27 after the needle cover is inserted into the reservoir unit housing;
- Fig. 29: the reservoir unit of fig. 27 and 28 after the cap is attached to the reservoir unit housing;
- Fig. 30: the reservoir unit of fig. 27 to 29 after the reservoir is inserted into the reservoir unit housing;
- Fig. 31: the reservoir unit of fig. 27 to 29 after the needle cover is pushed in the distal direction;
- Fig. 32: detail XXX of fig. 29 in an enlarged view; and
- Fig. 33: detail XXXI of fig. 31 in an enlarged view.

The reference symbols used in the drawings, and their primary meanings, are listed in summary form in the list of designations. In principle, identical parts are provided with the same reference symbols in the figures.

### DETAILED DESCRIPTION OF THE PREFFERRED EMBODIMENTS

In the present description the term "distal" refers to the side where the injection needle is located. This is on the right side or the lower part of the figures. The term "proximal" refers to the opposite side or rear end of the device. This is on the left side or the upper part of the figures.

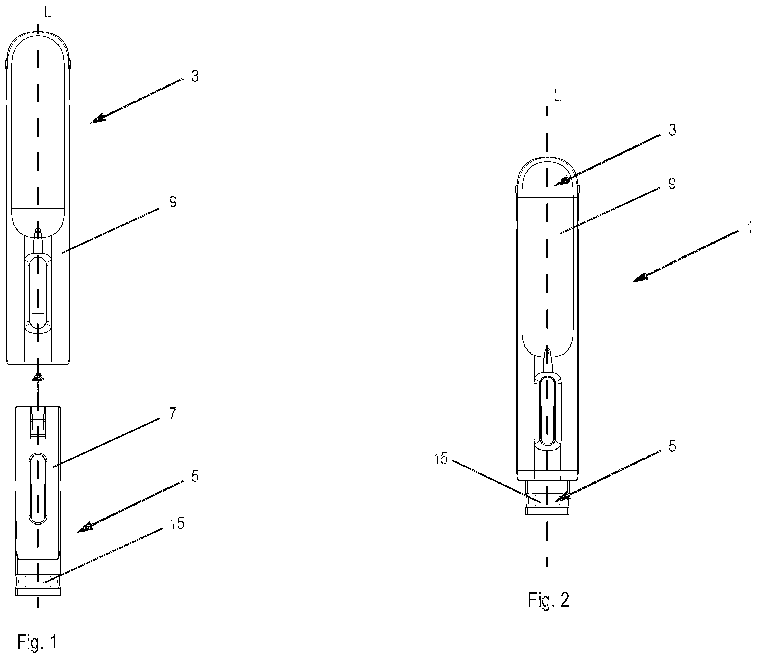

Fig. 1 to 10 show a first example embodiment of a drug delivery device 1 of the present disclosure in form of a semi-reusable autoinjector.

The autoinjector 1 comprises a reusable drive unit 3 and a disposable reservoir unit 5 insertable in the drive unit 3. The reservoir unit 5 is insertable into the drive unit 3 from a distal end of the drive unit 3. In the first example embodiment, the reservoir unit 5 is insertable by a linear movement in a longitudinal direction along a longitudinal axis L defined by the drive unit 3, as indicated by the arrow in fig. 1.

Fig. 1 shows the autoinjector before the reservoir unit 5 is inserted in the drive unit 3 and fig. 2 shows the fully assembled autoinjector 1 after the reservoir unit 5 is inserted into the drive unit 3. The drive unit 3 of this first example embodiment has a length of 160 mm in the longitudinal direction and an elliptical cross section with outer dimensions of 30.4 mm and 24.8 mm. The reservoir unit 5 of the first example embodiment has a length of 90.1 mm and a rectangular cross section with rounded edges and outer dimensions of 21.6 mm and 19.2 mm. The assembled autoinjector 1 as shown in fig. 2 has a total length of 175.6 mm, hence a major part of the reservoir unit 5 is inserted into the drive unit 3. This allows for a compact design of the autoinjector 1.

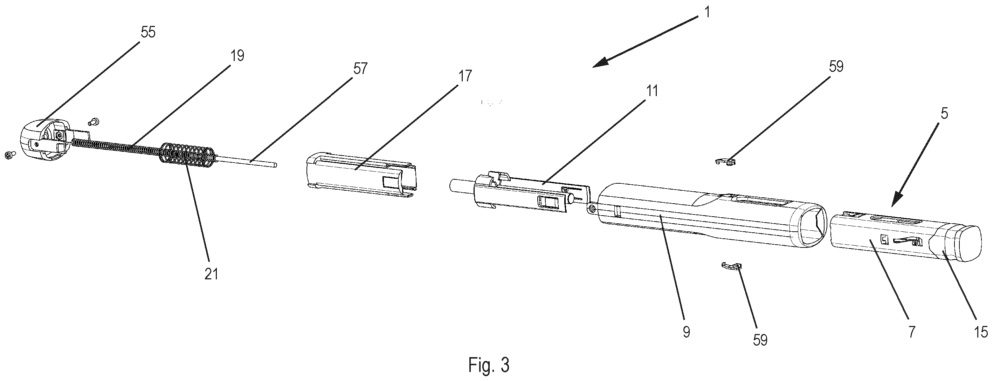

Fig. 3 shows the autoinjector 1 in an exploded view that gives an overview of the separate components of the drive unit 3 of the autoinjector 1. The main components of the drive unit 3 are a drive unit housing 9, a plunger 11, a pusher sleeve 17, a mechanical drive 19 in form of a compression spring and a pusher sleeve spring 21 also in form of a compression spring. In the first example embodiment, the drive unit housing 9 comprises a separate drive unit cap 55 that closes the drive unit housing 9 at a proximal end. The drive unit cap 55 is attachable to the drive unit housing 9 by a screw and nut connection and corresponding screw holes in the drive unit housing 9 and the drive unit cap 55. In other embodiments, the drive unit cap can also be formed integral with the rest of the drive unit housing. The drive unit 3 also comprises a mechanical drive rod 57 that is arranged inside the mechanical drive 19. The mechanical drive rod 57 prevents the mechanical drive 19 from bending during compression and relaxation of the mechanical drive 19. Further, the drive unit 3 comprises two emergency eject buttons 59, that allow the user to remove the reservoir unit 5 from the drive unit 3 at any time by pressing the emergency eject buttons 59.

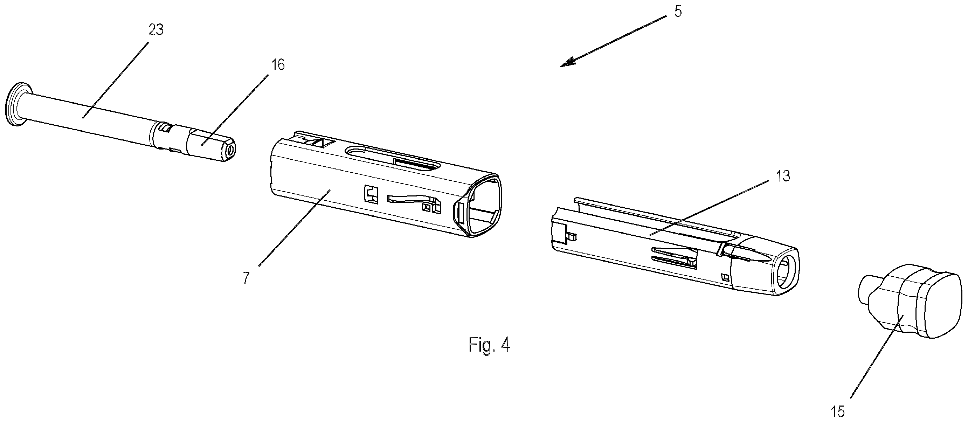

Fig. 4 shows the reservoir unit 5 of the autoinjector 1 in an exploded view that gives an overview of the separate components of the reservoir unit 5. The reservoir unit 5 comprises a reservoir unit housing 7, a needle cover 13, a cap 15 and a reservoir 23 with a needle. The reservoir 23 is a prefilled syringe or cartridge with a stopper at a proximal end and the needle permanently attached to a distal end. The reservoir 23 further comprises a needle shield 16 that covers and protects the needle and that has to be removed before the injection.

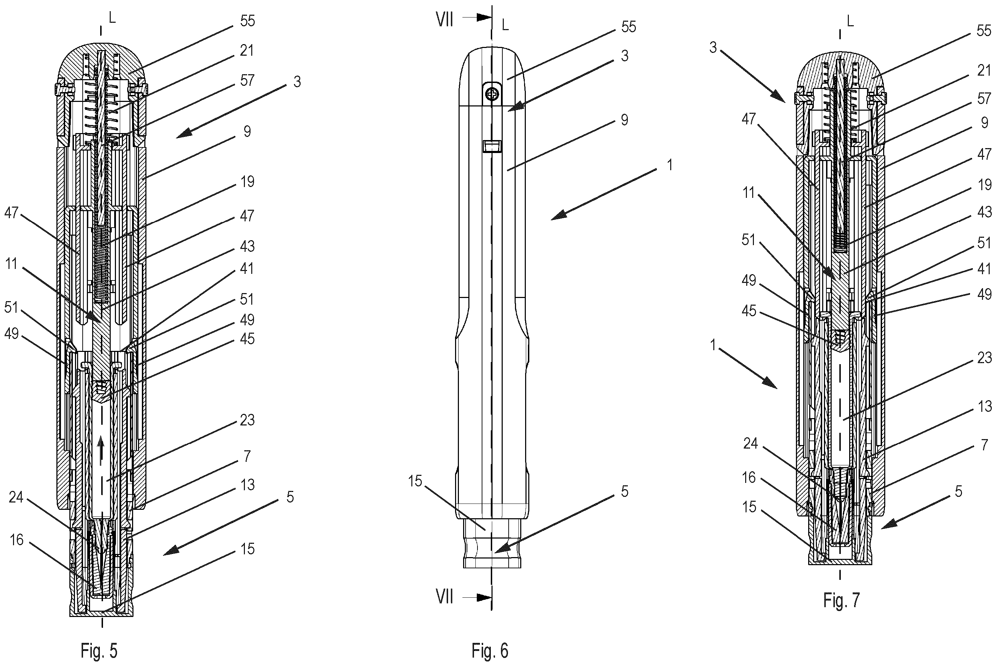

Fig. 5 shows the drive unit 3 and the reservoir unit 5 in a cross-sectional view while the reservoir unit 5 is inserted into the drive unit housing 9, as indicated by the arrow in fig. 5. While the reservoir unit 5 is inserted into the drive unit housing 9, a proximal end surface of the reservoir unit housing 7 contacts the plunger 11 and pushes the plunger 11 in the proximal direction to load the mechanical drive 19 by compressing the compression spring. The proximal end surface of the reservoir unit housing 7 therefore forms an abutment surface 41 arranged to contact the plunger 11. The plunger 11 comprises an inner rod section 43 with a distal end surface adapted to contact and move the stopper 45 of the reservoir 23 for dispensing the liquid drug and two plunger arms 47 arranged radially offset from the inner rod section 43 adapted to contact the abutment surface 41. The inner rod section 43 has a sleeve-shaped proximal section that defines an interior adapted to receive the mechanical drive 19 therein and forms a proximal end surface adapted to serve as contact surface for the distal end of the mechanical drive 19. Each plunger arm 47 comprises a plunger clip 49 with a projection 51 on its free end. When the reservoir unit 5 is inserted into the drive unit housing 9, the abutment surface 41 contacts the projections 51 on the plunger clips 49 to move the plunger 11 in the proximal direction. The plunger clips 49 further serve to lock the plunger 11 before the injection is started and to release the plunger 11 in order to start the injection, which will be explained in detail further below. In the first embodiment, the plunger clips 49 are located on a distal end of the plunger arms 47 and the projections 51 are located on a free proximal end of the plunger clips 49. The distal end of each plunger clip 49 connects the plunger clip 49 to the respective plunger arm 47.

Fig. 6 to 9 show the fully assembled autoinjector 1 when the reservoir unit 5 has reached a predefined end position within the drive unit housing 9. As can be seen from a comparison of fig. 5 and 7, by inserting the reservoir unit 5 into the drive unit housing 9, the reservoir unit 5 is moved to a predefined proximal position or end position within the drive unit housing 9 and thereby pushes the plunger 11 in the proximal direction with the abutment surface 41. The end position corresponds to a loaded state of the mechanical drive 19 due to a compression of the mechanical drive 19 between a proximal inner end of the drive unit cap 55 as part of the drive unit housing 9 and the proximal end surface of the inner rod section 43 of the plunger 11.

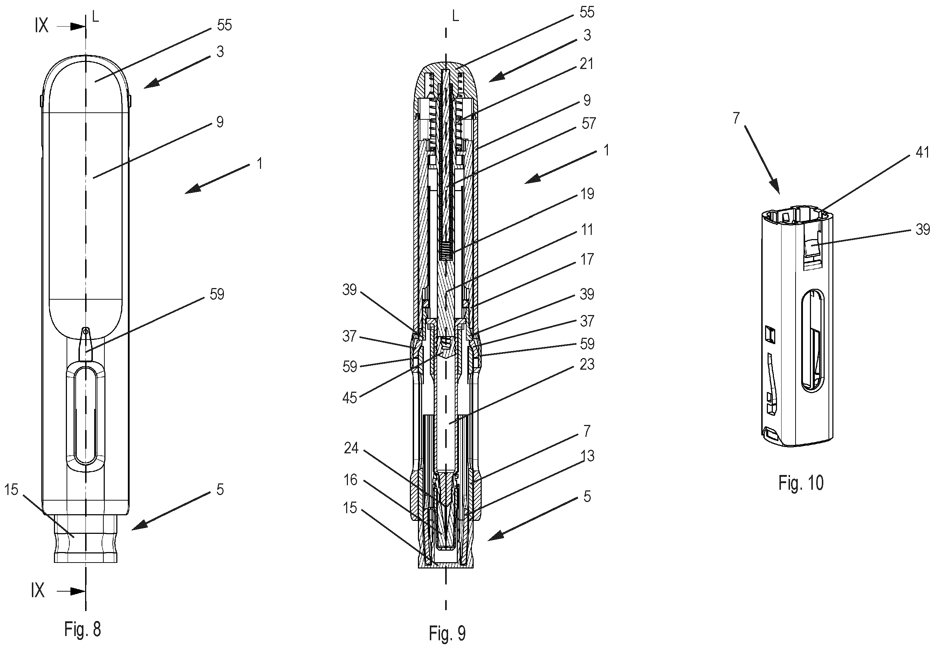

As can be seen from fig. 9, in the end position of the reservoir unit 5, the reservoir unit 5 is held in place against the force of the loaded mechanical drive 19 by a connection established by a first connection element 37 of the drive unit housing 9 and a second connection element 39 of the reservoir unit housing 7 that are in contact with each other. The first connection element 37 is formed by two protrusions on an inner surface of the drive unit housing 9 opposite to each other and the second connection element 39 is formed by two elastically deformable reservoir clips opposite to each other and near the proximal end of the reservoir unit housing 7. Fig. 10 shows the reservoir unit housing 7 with the elastically deformable reservoir clips 39 in detail. During the insertion, the reservoir clips 39 first approach the protrusions 37 and then pass the protrusions 37. When the reservoir unit 5 and the drive unit 3 are connected by the connection elements 37, 39, the plunger 11 is held by means of the projections 51 abutting the abutment surface 41, so that the plunger 11 cannot move in the distal direction to dispense the medicament from the reservoir 23. As shown in fig. 7, in the predefined end position of the reservoir unit 5, the needle cover 13 engages with the pusher sleeve 17 of the drive unit 3.

Before the user can use the autoinjector 1 to make an injection, the user has to remove the cap 15 from the autoinjector 1 by pulling on the cap 15 in the distal direction or in other embodiments by turning the cap. The cap 15 is engaged with the needle shield 16 of the reservoir 23, so that when the cap 15 is removed, the needle shield 16 is removed together with the cap 15.

The connection between the first connection elements 37 and the second connection elements 39 is released automatically by the pusher sleeve 17 after the injection is completed or can be manually released at any time by pressing the emergency eject buttons 59. The emergency eject buttons 59 are necessary for example when the injection fails to complete or if the wrong reservoir unit 5 is inserted into the drive unit 3 and the user wants to change the reservoir unit 5 or when the user stops the injection before the full dose of the medicament is dispensed. A wrong reservoir unit 5 can for example be a reservoir unit 5 containing a wrong or expired medicament.

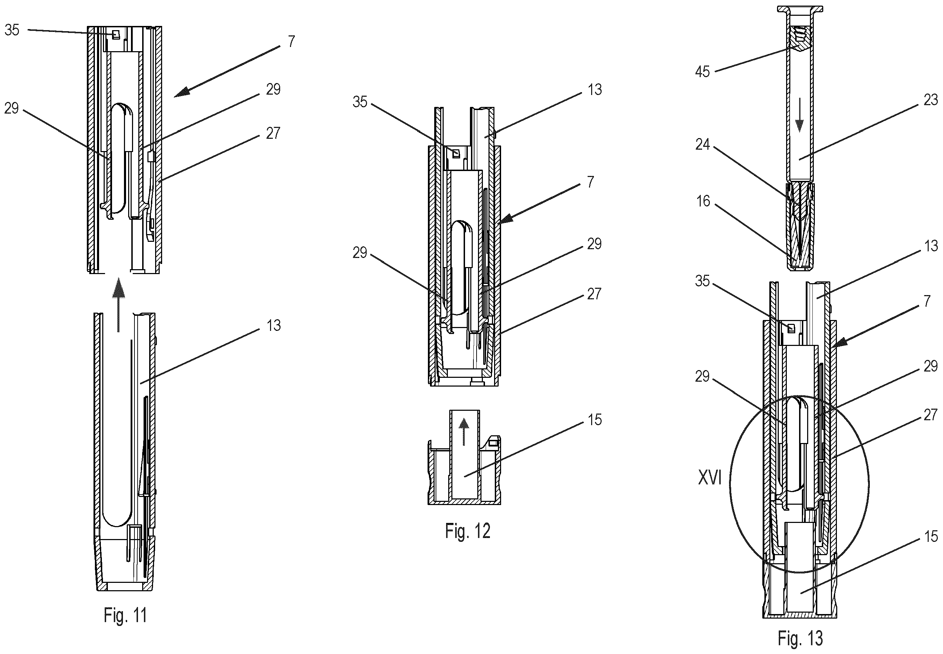

Fig. 11 to 17 show a first example embodiment of a reservoir unit 5 of the present disclosure that can be inserted into the drive unit 3 of the first example embodiment of the autoinjector 1 in fig. 1 to 10.

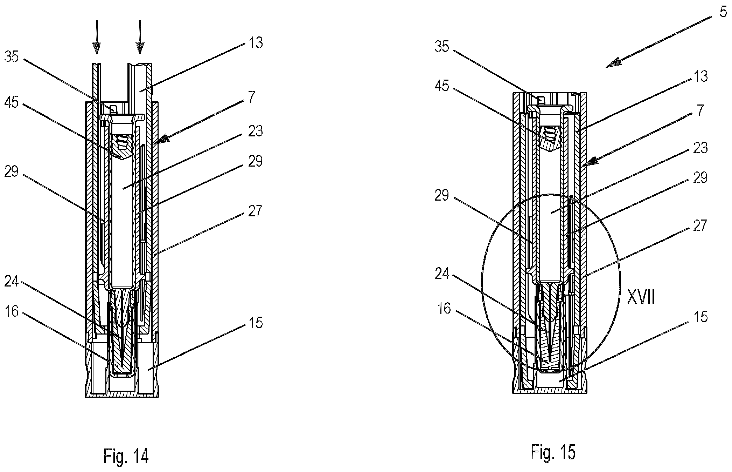

Fig. 11 shows the reservoir unit housing 7 and the needle cover 13 before assembly. The reservoir unit housing 7 comprises an outer sleeve section 27 and two inner reservoir arms 29. In a first assembly step, the needle cover 13 is inserted into the reservoir unit housing 7 from a distal end of the reservoir unit housing 7 by a linear movement in the longitudinal direction of the reservoir unit 5, as indicated by the arrow in fig. 11. As can be seen from fig. 12, the needle cover 13 is inserted between the outer sleeve section 27 and the reservoir arms 29, hence the sleeve-shaped wall of the needle cover 13 is arranged between the wall of the outer sleeve section 27 and the reservoir arms 29. After the insertion, the needle cover 13 protrudes from the proximal end of the reservoir unit housing 7. In a second assembly step, a cap 15 is attached to the reservoir unit housing 7 from the distal end of the reservoir unit housing 7 as indicated by the arrow in fig. 12. In a third assembly step, the reservoir 23 is inserted into the reservoir unit housing 7 from a proximal end of the reservoir unit housing 7, hence from the other side of the reservoir unit housing 7 than the needle cover 13 and the cap 15, as indicated by the arrow in fig. 13. This means the reservoir 23 is the last part to be assembled into the reservoir unit housing 7. As can be seen from fig. 14, the reservoir 23 is arranged inside the reservoir arms 29. In a fourth assembly step, the needle cover 13 is pushed in the distal direction, as indicated by the arrows in fig. 14, so that the proximal end of the needle cover 13 is on level with the proximal end of the reservoir unit housing 7. This fixes the reservoir 23 inside the reservoir arms 29, so that the reservoir 23 cannot move in the distal direction relative to the reservoir unit housing 7.

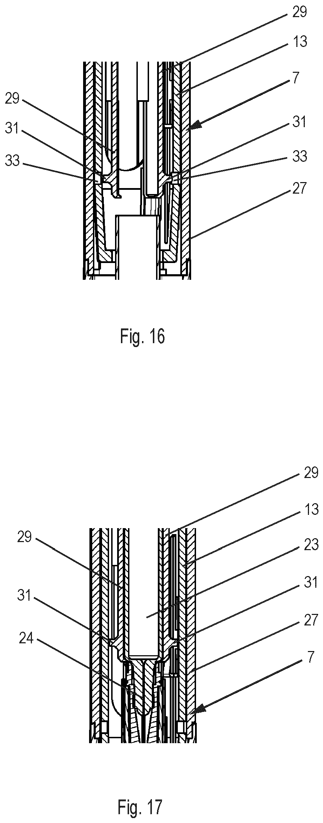

By a comparison of fig. 16 and 17 it is visible how the reservoir 23 is fixed inside the reservoir arms 29. The reservoir arms 29 each comprise a protrusion 31 on an outer surface of the reservoir arms 29 near the distal end of the reservoir arms 29. Before the needle cover 13 is pushed in the distal direction, as shown in fig. 14 and 16, the protrusions 31 are arranged in corresponding recesses 33 of the needle cover 13, so that the reservoir arms 29 are free to move in the radial direction, which allows the reservoir 23 to be inserted without the need of a high insertion force. Then, after the needle cover 13 is pushed in the distal direction, the recesses 33 are spaced apart from the protrusions 31 in the distal direction, as shown in fig. 15 and 17. Therefore, the protrusions 31 abut an inner surface of the needle cover 13 next to the recesses 33, so that the reservoir arms 29 cannot move in the radial direction. This fixes the reservoir 23 inside the reservoir arms 29.

To prevent the reservoir 23 from moving in the proximal direction with respect to the reservoir unit housing 7, the reservoir unit housing 7 comprises a bump feature 35 in form of a protrusion near a proximal end of the reservoir unit housing 7, as shown in fig. 11 to 15. The bump feature 35 abuts the proximal end of the reservoir 23 after the reservoir 23 is inserted.

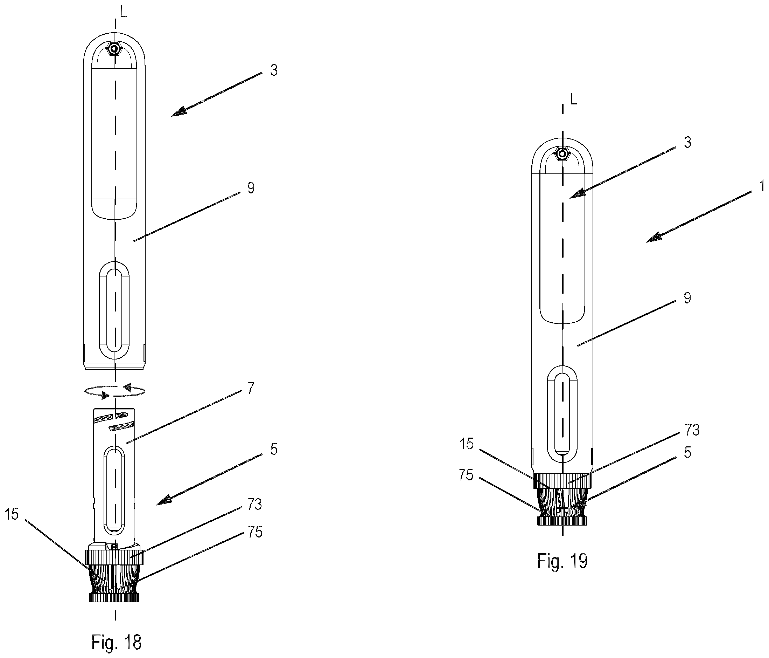

Fig. 18 to 26 show a second example embodiment of a drug delivery device 1 according to the present disclosure in form of a semi-reusable autoinjector.

The autoinjector 1 also comprises a reusable drive unit 3 and a disposable reservoir unit 5 insertable into the drive unit 3. The reservoir unit 5 is also insertable into the drive unit 3 from a distal end of the drive unit 3 but differs from the autoinjector 1 of fig. 1 to 10 in that the reservoir unit 5 is insertable by screwing the reservoir unit 5 into the drive unit 3. This means the reservoir unit 5 is insertable by a turning movement around the longitudinal axis L of the drive unit 3 as indicated by the arrow in fig. 18.

Fig. 18 shows the autoinjector 1 before the reservoir unit 5 is inserted into the drive unit 3 and fig. 19 shows the fully assembled autoinjector 1 after the reservoir unit 5 is inserted into the drive unit 3. The drive unit 3 of the second example embodiment has length of 159 mm in the longitudinal direction and an elliptical cross section with outer dimensions of 28 mm and 29 mm, hence and almost circular cross section. The reservoir unit 5 of the second example has a length of 90.3 mm and a circular cross section with a diameter of 27.2 mm. The assembled autoinjector 1 as shown in fig. 19 has a total length of 182.5 mm, hence a major part of the reservoir unit 5 is inserted into the drive unit 3. This allows for a compact design of the autoinjector 1.

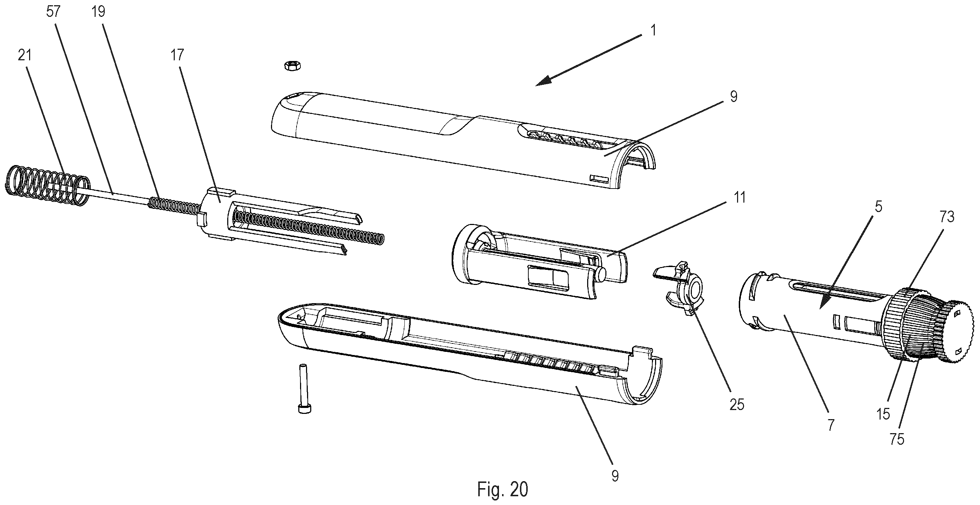

Fig. 20 shows the autoinjector 1 in an exploded view that gives an overview of the separate components of the drive unit 3 of the autoinjector 1. The main components of the drive unit 3 are a drive unit housing 9, a plunger 11, a pusher sleeve 17, a mechanical drive 19 in form of a compression spring, a pusher sleeve spring 21 also in form of a compression spring and a gear unit 25. The gear unit 25 rotates during the injection and provides a lockout of the needle cover 13 in a final extended position of the needle cover 13 in which the needle is fully covered by the needle cover 13. In the second example embodiment, the drive unit housing 9 comprises two housing parts that are connectable to each other by latches on the distal end of the drive unit housing 9 and a screw and nut connection on a proximal end of the drive unit housing 9. The drive unit 3 also comprises a mechanical drive rod 57 similar to the mechanical drive rod of the first example embodiment.

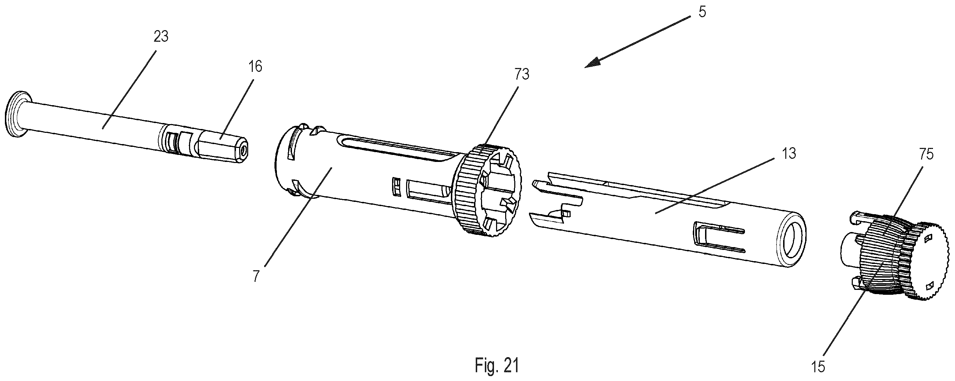

Fig. 21 shows the reservoir unit 5 of the autoinjector 1 in an exploded view that gives an overview of the separate components of the reservoir unit 5. The reservoir unit 5 comprises a reservoir unit housing 7, a needle cover 13, a cap 15 and a reservoir 23 with an injection needle covered by a needle shield 16.

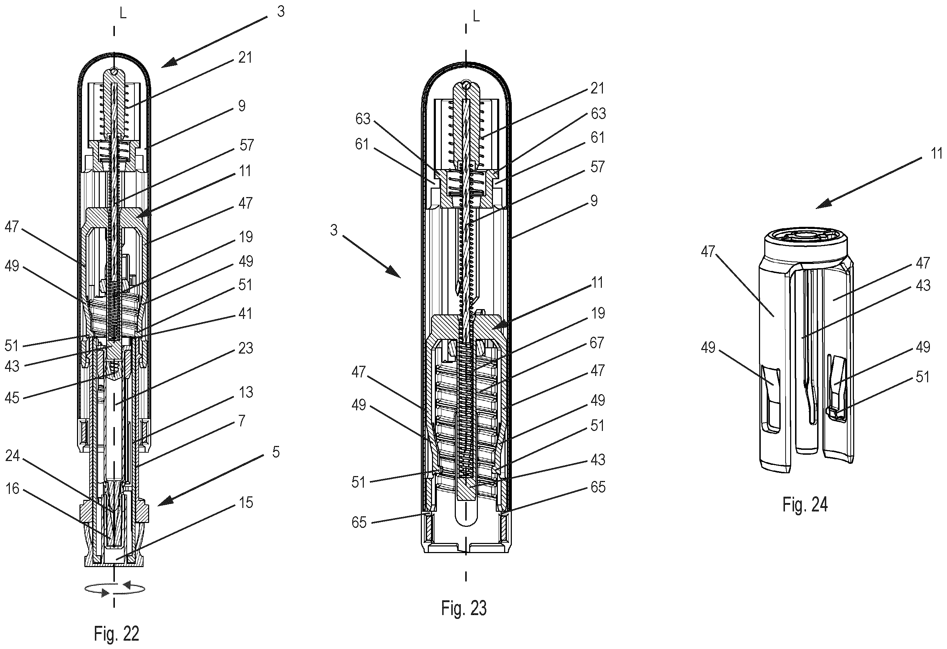

Fig. 22 shows the drive unit 3 and the reservoir unit 5 in a cross-sectional view while the reservoir unit 5 is inserted into the drive unit housing 9 by screwing the reservoir unit 5 into the drive unit housing 9, as indicated by the arrow in fig. 22. Therefore, the drive unit housing 9 comprises a thread 67 and the reservoir unit housing 7 comprises a thread 69 that are compatible to each other. The thread 67 of the drive unit housing 9 can comprise two starts to make it easier to insert the reservoir unit 5 into the drive unit housing 9, because the reservoir unit 5 can be inserted in two different orientations instead of just one orientation. Like for the first autoinjector 1 of figures 1 to 10, while the reservoir unit 5 is inserted into the drive unit housing 9, a proximal end surface of the reservoir unit housing 7 contacts the plunger 11 and pushes the plunger 11 in the proximal direction to load the mechanical drive 19 by compressing the compression spring. The proximal end surface of the reservoir unit housing 7 therefore forms an abutment surface 41 arranged to contact the plunger 11. During the insertion of the reservoir unit 5, the abutment surface 41 moves to a predefined proximal position or end position within the drive unit housing 9, which may correspond to a loaded state of the mechanical drive 19 due to compression of the mechanical drive 19 between the proximal end of the drive unit housing 9 and the proximal end surface of the inner rod section 43 of the plunger 11.

The plunger 11 is formed similar to the plunger 11 of the first autoinjector 1, as shown inf fig. 24 which shows the plunger 11 with the inner rod section 43 and the plunger arms 47 in detail. Different to the first example embodiment, in the second example embodiment the projections 51 are located on a free distal end of the plunger clips 49 instead of a free proximal end of the plunger clips 49. Hence, instead of the distal end of the plunger clips 49, the proximal end of the plunger clips 49 connects the plunger clips 49 to the plunger arms 47.

Fig. 23 shows the drive unit 3 in its initial state before the reservoir unit 5 is inserted. The pusher sleeve 17 is prevented from moving in the distal direction by circumferential proximal projections 61 on an inner surface of the drive unit housing 9 that abut a circumferential shoulder 63 of the pusher sleeve 17 and thereby ensure that the pusher sleeve 17 stays inside the drive unit housing 9. The plunger 11 is held inside the drive unit housing 9 and prevented from moving in the distal direction by the plunger arms 47 contacting a circumferential distal projection 65 of the drive unit housing 9. In the second example embodiment, the drive unit 3 can be assembled in that the plunger 11 and the pusher sleeve 17 are placed inside the drive unit housing 9 and after that the two housing halves are connected to each other.

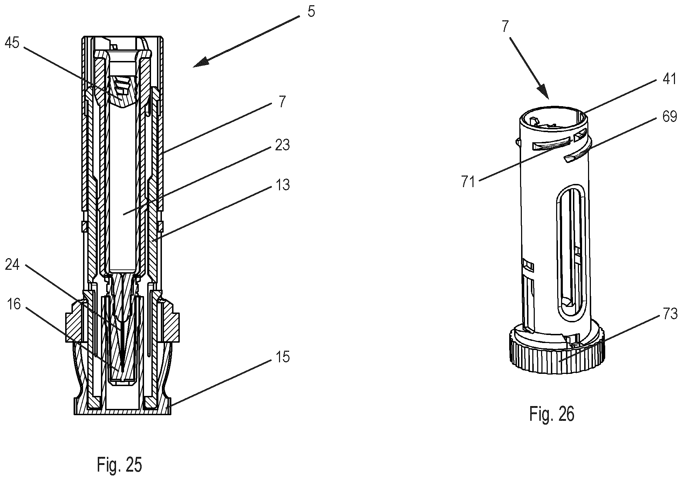

Fig. 25 shows the reservoir unit housing 5 in its initial state before it is inserted into the drive unit 3. In the second example embodiment, the cap 15 is locked to the reservoir unit housing 7, until the reservoir unit 5 is inserted into the drive unit housing 9 and has reached its predefined end position within the drive unit housing 9. The needle cover 13 is held in place inside the reservoir unit housing 7 by friction between the needle cover 13 and the reservoir unit housing 7. In other embodiments the reservoir unit housing and/or the needle cover could comprise an additional detent to keep the needle cover in its position. Fig. 26 shows the reservoir unit housing 7 with the abutment surface 41 and the thread 69 in detail. Insertion of the reservoir unit 5 into the distal end of the drive unit housing 9 followed by screwing the reservoir unit 5 into the drive unit housing 9 engages the thread 69 of the reservoir unit housing 7 with the complementary thread 67 of the drive unit housing 9. To make the screwing of the reservoir unit 5 in and out of the drive unit housing 9 easier, the reservoir unit housing 7 comprises a grip area 73 at a distal end of the reservoir unit housing 7 that the user can grip and hold easily and securely. To further facilitate the screwing of the reservoir unit 5, the cap 15 also comprises a grip area 75, so that the grip area is bigger, and it is easier and more comfortable for the user to grip the reservoir unit 5.

In the predefined end position the reservoir unit 5 and the drive unit 3 can be connected by a detent in the thread 67 of the drive unit housing 9 and a corresponding gap 71 in the thread 69 of the reservoir unit housing 7, as shown in fig. 26. To release the reservoir unit 5 from the drive unit 3, the user has to manually screw the reservoir unit 5 out of the drive unit housing 9 after the injection is complete.

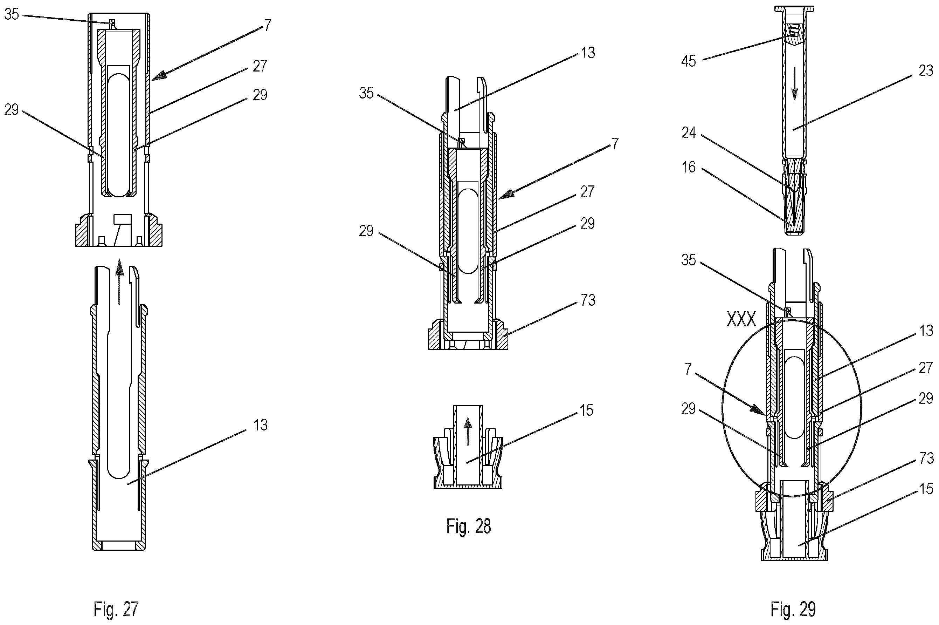

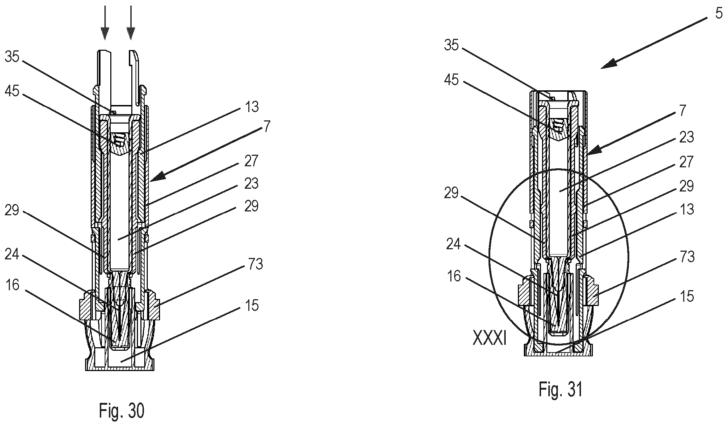

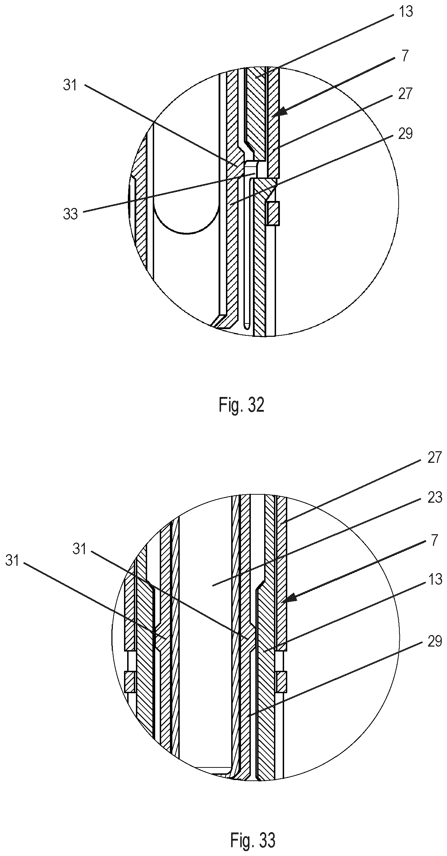

Fig. 27 to 33 show a second example embodiment of a reservoir unit 5 of the present disclosure that can be inserted into the drive unit 3 of the second example embodiment of the autoinjector 1 in fig. 18 to 26. The assembly process and the features of the separate components of the second example embodiment of the reservoir unit 5 are substantially the same as for the first reservoir unit 5 described above in connection with fig. 11 to 17, and accordingly only the differences between the two example embodiments are described herein.

In the second example embodiment, the protrusions 31 of the reservoir unit housing 7 are also arranged in corresponding recesses 33 of the needle cover 13 when the needle cover is in its proximal position. A proximal end surface of the protrusions 31 and a proximal end surface of the recesses 33 each include a ramp shape. During movement of the needle cover 13 in the distal direction, these ramp shaped surfaces facilitate movement of the needle cover 13 past the recesses 33, e.g., for a sliding movement. In addition, the arrangement of the protrusions 31 and corresponding recesses 33 in fig. 27 to 33 is more proximal relative to the arrangement of the protrusions 31 and corresponding recesses 33 in fig. 11 to 17 e.g., at a central region of the reservoir 23 when inserted. Thus, fixing the reservoir 23 inside the reservoir arms 29 due to the protrusions 31 abutting an inner surface of the needle cover 13 may occur along a different portion of the reservoir 23 compared to the first example embodiment.

It will be appreciated that in both example embodiments the reservoir unit housing 7 and the needle cover 13 may include more than one set of protrusions and recesses, and that the protrusions may alternatively be provided on the needle cover 13, e.g., inwardly facing protrusions, and that the cooperating recesses may alternatively be provided on the reservoir arms 29.

In use, for both embodiments of the autoinjector 1, the autoinjector 1 is ready to perform an injection after the user has removed the cap 15 from the autoinjector 1. To start the injection, the user presses the autoinjector 1 against the skin at a desired injection site. This causes the needle cover 13 to move from the initial position to the retracted position by moving in the proximal direction with respect to the reservoir unit housing 7 and the reservoir 23. While the needle cover 13 moves in the proximal direction, the needle cover 13 elastically deforms the plunger clips 49 in the radial direction from the initial position in which the plunger clips 49 hold the plunger 11 against the bias of the loaded mechanical drive 19 to a deformed position. In the deformed position, the plunger 11 is released so that the plunger 11 is free to move in the distal direction under the bias of the loaded mechanical drive 19 to start the injection and to expel the medicament from the reservoir 23. The trigger point for the release of the plunger 11 is shortly before the needle cover 13 reaches its retracted position or proximal position. After the injection, the needle cover 13 is moved to a final extended position that is distal from the initial position of the needle cover 13, hence the needle cover 13 protrudes further from the distal end of the reservoir unit housing 7 than in the initial position. In the final extended position, the needle cover 13 is locked out, hence it cannot move back in the proximal direction after the needle cover 13 has reached the final extended position.

The previous description of the disclosed embodiments is provided to enable a person skilled in the art to make or use the disclosed embodiments. Various modifications to these embodiments will be readily apparent to those skilled in the art, and the principles defined herein may be applied to other embodiments without departing from the scope of the disclosure. Thus, the present disclosure is not intended to be limited to the embodiments shown herein but is to be accorded the widest scope possible consistent with the principles and novel features as previously shown and described.

### LIST OF DESIGNATIONS

- 1: drug delivery device
- 3: drive unit
- 5: reservoir unit
- 7: reservoir unit housing
- 9: drive unit housing
- 11: plunger
- 13: needle cover
- 15: cap
- 16: needle shield
- 17: pusher sleeve
- 19: mechanical drive
- 21: pusher sleeve spring
- 23: reservoir
- 24: needle
- 25: gear assembly/gear unit
- 27: outer sleeve section
- 29: reservoir arm
- 31: protrusion
- 33: recess
- 35: bump feature
- 37: first connection element
- 39: second connection element
- 41: abutment surface
- 43: inner rod section plunger
- 45: stopper
- 47: plunger arm
- 49: plunger clip
- 51: projection
- 55: drive unit cap
- 57: mechanical drive rod
- 59: emergency eject button
- 61: proximal projection
- 63: shoulder
- 65: distal projection
- 67: thread
- 69: thread
- 71: gap
- 73: grip area
- 75: grip area

- L: longitudinal axis

## Claims

1. A loading mechanism for a drug delivery device (1) for dispensing a liquid drug from a reservoir (23) through a needle (24), the loading mechanism comprising a drive unit (3) and a reservoir unit (5) adapted to hold the reservoir (23) and releasably attachable to the drive unit (3),
the drive unit (3) comprising a drive unit housing (9) defining a longitudinal axis (L), a plunger (11) movable inside the drive unit housing (9) in a dispensing direction to dispense the liquid drug and a mechanical drive (19), preferably a compression spring, arranged inside the drive unit housing (9) for moving, when loaded, the plunger (11) in the dispensing direction,
the reservoir unit (5) comprising a reservoir unit housing (7),
wherein for loading the drug delivery device (1), the reservoir unit (5) is insertable into the drive unit housing (9) from a distal end of the drive unit (3),
**characterized in that** the reservoir unit housing (7) comprises an abutment surface (41) that is adapted to contact the plunger (11) and to push the plunger (11) in a proximal direction to load the mechanical drive (19), preferably to compress or re-set the compression spring, while the reservoir unit (5) is inserted into the drive unit housing (9).

2. Loading mechanism according to claim 1, wherein the plunger (11) comprises an inner rod section (43) adapted to contact and move a stopper (45) of the reservoir (23) for dispensing the liquid drug and a plunger arm (47) arranged radially offset from the inner rod section (43) adapted to contact the abutment surface (41).

3. Loading mechanism according to claim 2, wherein the plunger arm (47) comprises a plunger clip (49) that is elastically deformable in a radial direction and adapted to hold the plunger (11) in an initial position against a bias of the loaded mechanical drive (19) and to release the plunger (11) in a deformed position.

4. Loading mechanism according to claim 2 or 3, wherein the plunger arm (47) comprises a projection (51) on an inner surface of the plunger arm (47) that is adapted to be contacted by the abutment surface (41) of the reservoir unit housing (7), wherein preferably the projection (51) is located on the plunger clip (49).

5. Loading mechanism according to claim 4, wherein the reservoir unit housing (7) is engaged with the drive unit housing (9) when inserted and the plunger (11) is held by means of the projection (51) abutting the abutment surface (41).

6. Loading mechanism according to one of claims 3 to 5, wherein for releasing the plunger (11), the plunger clip (49) is deformable by a needle cover (13) of the reservoir unit (5).

7. Loading mechanism according to one of the preceding claims, wherein the reservoir unit (5) is insertable into the drive unit housing (9) by a linear movement along the longitudinal axis (L).

8. Loading mechanism according to claims 3, 4 and 7, wherein the plunger clip (49) is located on a distal end of the plunger arm (47) and the projection (51) is located on a free proximal end of the plunger clip (49).

9. Loading mechanism according to one of claims 1 to 6, wherein the reservoir unit (5) is insertable into the drive unit housing (9) by a turning movement around the longitudinal axis (L), preferably by screwing the reservoir unit (5) into the drive unit housing (9).

10. Loading mechanism according to claims 3, 4 and 9, wherein the plunger clip (49) is located on a distal end of the plunger arm (47) and the projection (51) is located on a free distal end of the plunger clip (49).

11. Reservoir unit (5) for a loading mechanism according to one of claims 1 to 10, comprising the reservoir unit housing (7), the reservoir (23) and a needle cover (13), wherein the reservoir unit housing (7) comprises an outer sleeve section (27) and two inner reservoir arms (29) arranged inside the outer sleeve section (27), **characterized in that** the reservoir unit (5) is adapted to be assembled in the following way:
- the needle cover (13) is insertable into the reservoir unit housing (7) from a distal end of the reservoir unit housing (7), wherein the needle cover (13) is insertable between the reservoir arms (29) and the outer sleeve section (27);
- after the needle cover (13) is inserted, the reservoir (23) is insertable into the reservoir unit housing (7) from a proximal end of the reservoir unit housing (7), wherein the reservoir (23) is insertable inside the reservoir arms (29);
- after the reservoir (23) is inserted, the needle cover (13) is movable in a distal direction from a first position in which the reservoir arms (29) are free to move in a radial direction to a second position in which the needle cover (13) prevents the reservoir arms (29) from moving in the radial direction, so that the reservoir (23) is fixed against distal movement inside the reservoir arms (29).

12. Reservoir unit (5) according to claim 11, wherein the reservoir arms (29) each comprise a protrusion (31) facing the outer sleeve section (27) and the needle cover (13) comprises two recesses (33) adapted to receive the protrusions (31) in the first position of the needle cover (13).

13. Reservoir unit (5) according to claim 12, wherein the protrusions (31) are located near a distal end of the reservoir arms (29).

14. A drug delivery device (1) comprising a loading mechanism according to one of claims 1 to 10, wherein in particular the reservoir unit housing (7), the drive unit housing (9) and/or the plunger (11) are injection-molded thermoplastic plastic parts.

15. Drug delivery device (1) according to claim 14 with a reservoir unit (5) according to one of claims 11 to 13, wherein in particular the needle cover (13) is an injection-molded thermoplastic plastic part.
